# EUROPÄISCHE PATENTSCHRIFT

(11) **EP 1 441 035 B1**
(45) Veröffentlichungstag und Bekanntmachung des Hinweises auf die Patenterteilung: **23.03.2005**
(21) Anmeldenummer: 03001524.2
(22) Anmeldetag: 23.01.2003
(51) Int. Cl.: C12Q 1/68, G01N 33/68

(54) **Verfahren zur Erstellung von Profilen der differentialen Proteinexpression**
Method of obtaining profiles of differential protein expression
Procédé pour obtenir des profiles d'expression différentielle de protéines

(43) Veröffentlichungstag der Anmeldung: 28.07.2004
(73) Patentinhaber: BioVendor Laboratory Medicine, Inc., 612 00 Brno (CZ)
(72) Erfinder: Ruzicka, Viktor, 616 00 Brno (CZ)
(74) Vertreter: Baumbach, Friedrich, Dr.

(56) Entgegenhaltungen:
- WO-A-96/33406
- GHARBI SEVERINE ET AL: "Evaluation of two-dimensional differential gel electrophoresis for proteomic expression analysis of a model breast cancer cell system." MOLECULAR & CELLULAR PROTEOMICS, Bd. 1, Nr. 2, Februar 2002 (2002-02), Seiten 91-98, XP002243191 February, 2002 ISSN: 1535-9476
- TAKESHI SANO ET AL: "IMMUNO-PCR: VERY SENSITIVE ANTIGEN DETECTION BY MEANS OF SPECIFIC ANTIBODY-DNA CONJUGATES" SCIENCE, AMERICAN ASSOCIATION FOR THE ADVANCEMENT OF SCIENCE,, US, Bd. 258, Nr. 5079, 2. Oktober 1992 (1992-10-02), Seiten 120-122, XP000384402 ISSN: 0036-8075
- ADAM GREGORY C ET AL: "Chemical strategies for functional proteomics." MOLECULAR & CELLULAR PROTEOMICS, Bd. 1, Nr. 10, 20. Oktober 2002 (2002-10-20), Seiten 781-790, XP009011850 ISSN: 1535-9476
- YOKOTA H ET AL.: "Elucidation of the mechanism of action of a proprietary compound on a liver cell line using 2-D DIGE" LIFE SCIENCE NEWS, Nr. 12, 2002, Seiten 1-3, XP002243192

## Beschreibung

Die Erfindung betrifft ein Verfahren zur Erstellung von Profilen der differentiellen Proteinexpression unter Verwendung von spezifischer Nukleinsäuren-Markierung des zu untersuchenden Proteoms und quantitativer Amplifizierung nach Auftrennung der markierten Proteine. Anwendungsgebiete sind die Medizin und die Molekularbiologie.

Eine Hauptaufgabe der Molekularbiologie nach der Entschlüsselung des menschlichen Genoms ist die Identifizierung der Sequenzen, die als Bauplan für Proteine Verwendung finden und vor allem die Funktionen und Regulationsmechanismen der Proteine zu analysieren. In letzter Zeit wurden viele Bemühungen unternommen, die Aufgaben von Genen und den daraus resultierenden Proteinen zu untersuchen. Dabei wurden vor allem mRNA Expressionsprofile und subzelluläre Lokalisationen untersucht. Obwohl diese Studien sehr nützlich sind, korrelieren die Expressionsprofile auf mRNA Ebene nicht notwendigerweise gut mit den zellulären Protein-Levels. Außerdem sind Methoden, die auf mRNA- und/oder cDNA-Ebene durchgeführt werden, in zellfreien Systemen wie Blutserum, Urin oder CSF nicht anwendbar.

Die Untersuchung von Expressionsprofilen auf Proteinebene ist deshalb besonders wichtig. Techniken, die bisher dafür verwendet werden, sind die zweidimensionale Elektrophorese, die _{"}free flow electrophorese" mit einem pH-Gradienten in der Querrichtung, die im Wesentlichen eine Art präparative isoelektrische Fokussierung darstellt und die Massenspektroskopie, gekoppelt an die zweidimensionale Elektrophorese oder eine chromatografische oder kapillar-elektrophoretische Auftrennung der Probe. Diese Methoden sind zwar zur Analyse der vorhandenen Proteine gut geeignet, jedoch ist es relativ schwierig und zum Teil sehr aufwendig, qualitative und/oder quantitative Veränderungen der Proteinexpression nach einer Zustandsänderung der diese Proteine produzierenden Zellen zu untersuchen. Eines der wichtigsten Probleme der oben aufgeführten Methoden ist eine limitierte Sensitivität, d.h. Proteine im unteren Atto Molbereich sind kaum nachweisbar. Gerade die regulatorischen Proteine, die für pharmakologische und diagnostische Zwecke untersucht werden oder Markerproteine kommen oft im unteren Atto-Molbereich vor.

WO 96/33406; Yokota, H. et al. (Life Science New, 12:1-3, 2002, Amersham Bioscience), Gharbi, S. et al. (Molecular & Cellular Proteomics, 1:91-98, 2002) und Adam, G.C. et al. (Moleuclar & Cellular Proteomics, 1:781-790) offenbaren Verfahren zur Erstellung von Profilen differentieller Proteinexpression, bei denen die zu vergleichenden Proteome mit unterschiedlichen Farbstoffen oder lsotopen markiert werden.

Der Erfindung lag somit die Aufgabe zugrunde, ein Verfahren zu entwickeln, mit dem Profile der Proteinexpression erstellt und qualitative und/oder quantitative Veränderungen der Proteinexpression analysiert werden können.

Die Erfindung wird gemäß den Ansprüchen realisiert.

Gegenstand der Erfindung ist ein Verfahren zur Erstellung von Profilen der differentiellen Proteinexpression zum Vergleich von Proteomen gemäß Anspruch 1.

Die Nukleinsäure-Sequenzen, die zur Markierung der Proteine verwendet werden, besitzen eine Länge von mindestens 45 Basenpaaren und bestehen aus identischen 3'- und 5'endständigen Sequenzen mit einer bevorzugten Länge von 15 bis 25 Basenpaaren und unterschiedlichen Sequenzabschnitten im mittleren Bereich mit einer Länge von mindestens 15, bevorzugt 20 Basenpaaren. Sie habe an einem Ende eine reaktive Gruppe, vorzugsweise eine SH2- oder NH2-Gruppe an einem 5'- oder 3'-Ende. Diese reaktiven Gruppen werden als Thiolink und Aminolink bezeichnet und können mit Hilfe sogenannter Crosslinker an eine NH₂-, COOH- oder SH-Gruppe eines Proteins kovalent gebunden werden.

Als Crosslinker werden bevorzugt N-Succinimidyl-(4-vinylsulfonyl)benzoate, Sulfosuccinimidyl-4-(P-Maleimidophenyl) Butyrate, Succinimidyl-4(N-maleimidomethyl) cyclohexane-1-carboxylate oder Sulfosuccinimidyl-4(N-maleimidomethyl)cyclohexane-1-carboxylate eingesetzt.

Als identische 3'- und 5'-endständige Sequenzen kommen alle Sequenzen in Betracht, die mit einem Primerpaar in der PCR gut funktionieren. Vorzugsweise werden folgende Sequenzen verwendet:

+Strang: 5´-GAGCATTCACGGTTCCTAAG.......................GAGTATCACGTTGCTAGTCG-3´
- Strang: 3´-CTCGTAAGTGCCAAGGATTC.......................CTCATAGTGCAACGATCAGC-5´.

Als unterschiedliche Sequenzabschnitte im mittleren Bereich können alle Sequenzen eingesetzt werden, die sich mit gleicher Amplifizierungskinetik amplifizieren lassen und mit einer bestimmten Sonde gut hybridisieren oder charakteristisches Schmelzverhalten aufweisen.

Vorzugsweise finden folgende Sequenzen Verwendung:

Sequenz A
+Strang: 5´-ACTTCACGTGCATTGGCACACTCGATGCAATTGATCAGGC
-Strang: 3´-TGAAGTGCACGTAACCGTGTGAGCTACGTTAACTAGTCCG

Sequenz B
+Strang: 5´-CAATACAGAGACTACGACACATAGCAGACCTAGCTACGGA
-Strang: 5´-GTTATGTCTCTGATGCTGTGTATCGTCTGGATCGATGCCT

Die Auftrennung der Proteine erfolgt vorzugsweise durch Chromatografie, wie Ionenaustausch, hydrophobe Interaktion, _{"}reverse phase", Permätionschromatografie, Immunaffinitäts-Chromatographie, Metall-Bindung-Chromatografie, Heparin-, Gelatineoder ähnliche Chromatografie sowohl chirale oder andere chromatografische Auftrennungsmethoden, sowie Kapillar-Elektrophorese, Isotachophorese, isoelektrische Fokussierung oder klassische Akrylamid- oder Agarose-Elektrophorese.

Die Isolierung der Proteine erfolgt vorzugsweise durch Erhöhung der Menge der aufgetragenen Protein-Mischung und konsequente Erhöhung der Protein-Menge in der herausgesuchten Fraktion mit anschließendem zweiten Auftrennungsverfahren und Sammeln der einzelnen Proteine oder sequenzspezifische Bindung der DNA-Markierung an eine immobilisierte Sonde.

Die quantitative Bestimmung der Nukleinsäure-Sequenzen erfolgt vorzugsweise durch quantitative PCR; wobei eine Unterscheidung zwischen den Sequenzen, mit denen das Proteom der Zellen oder zellfreier Systeme vor der Zustandsänderung markiert wurde und den Sequenzen, mit denen das Proteom der Zellen oder zellfreier Systeme nach der Zustandsänderung markiert wurde, durch die unterschiedlichen Sequenzabschnitte im mittleren Bereich ermöglicht wird.

Dabei werden die Nukleinsäure-Sequenzen (Amplifikation) während der quantitativen PCR mit Primem amplifiziert, die komplementär zu den identischen 3'- und 5'-endständigen Sequenzen sind und mit Hilfe von Sonden detektiert, die spezifisch an die unterschiedlichen Sequenzabschnitte im mittleren Bereich hybridisieren. Alternativ können charakteristische Schmelzkurven der jeweiligen Markierungs-Sequenzen alleine oder in Kombination mit den erwähnten Sonden die Zuordnung der Markierungs-Sequenz sicherstellen.

Als Sonden werden fluoreszenzmarkierte Sonden verwendet, bevorzugt markiert mit FAM, TAMRA, JOE, HEX, BIODIPY, Oregon Green, Rhodamine, Cy3, Cy5 oder CY5.5.

Anschließend erfolgt ein Vergleich der Mengen der gebundenen Nukleinsäure-Sequenzen der Zellen vor und nach der Zustandsänderung, wobei eine erhöhte Konzentration der Sequenzen, mit denen das Proteom der Zellen oder ein zellfreies Proteinsystem vor der Zustandsänderung markiert wurde gegenüber einer geringeren Konzentration der Sequenzen, mit denen das Proteom der Zellen oder ein zellfreies Proteinsystem nach der Zustandsänderung markiert wurde, eine Verringerung der Expression des untersuchten Proteins durch die spezielle Zustandsänderung anzeigt bzw. eine erniedrigte Konzentration der Sequenzen, mit denen das Proteom der Zellen oder ein zellfreies Proteinsystem vor der Zustandsänderung markiert wurde gegenüber einer höheren Konzentration der Sequenzen, mit denen das Proteom der Zellen oder ein zellfreies Proteinsystem nach der Zustandsänderung markiert wurde, eine Erhöhung der Expression des untersuchten Proteins durch die spezielle Zustandsänderung anzeigt.

Abschließend werden die Proteine durch gängige Methoden, vorzugsweise durch Massenspektroskopie, zweidimensionale oder eindimensionale Elektrophorese bzw. Aminosäuren-Sequenzierung identifiziert.

Kern der Erfindung ist somit ein Verfahren zur Erstellung von Profilen der differentialen Proteinexpression unter Verwendung einer spezifischen Nukleinsäuren-Markierung des zu untersuchenden Proteoms und quantitativer Amplifizierung dieser Markierung nach Auftrennung der markierten Proteine. Verwendung finden dabei zwei oder mehrere Nukleinsäure-Sequenzen zur kovalenten Markierung von Proteinen vor und nach einer (oder mehreren) Zustandsveränderung(en) der sie produzierenden Zellen oder eines zellfreien Proteinsystems, die zur qualitativen und/oder quantitativen Veränderung der Proteinexpression führ(t)en. Durch quantitative Amplifizierung dieser Markierungssequenzen nach Auftrennung der Proteine werden Veränderungen der Proteinexpression analysiert. Der große Vorteil des erfindungsgemäßen Verfahrens gegenüber den bisher verwendeten Verfahren ist, dass sich damit Proteine im Zeptomol-Bereich nachweisen lassen.

Im folgenden wird die Erfindung näher erläutert:

### 1. Markierung

Eine der Sequenzen (A) wird für die Markierung der Proteine vor der Zustandsveränderung (Proteinpool a) verwendet, die andere(n) (B, C...) für die Markierung der Proteine nach der Zustandsveränderung (Proteinpool b, ggf. c...). Die Sequenzen haben identische Endstrecken und unterscheiden sich in der internen Sequenz, so dass Amplifikate, die mit einem und demselben Primer-Paar hergestellt werden, voneinander unterscheidbar sind. Nach erfolgter Markierung des Proteinpools a (mit der Markierungssequenz A) und des Proteinpools b (c...) (mit der Markierungssequenz B (C...)) werden die markierten Proteinpools zusammengelegt und aufgetrennt.

### 2. Auftrennung

Die Auftrennung erfolgt so, dass die einzelnen jetzt markierten Proteine, gleich ob sie aus dem Proteinpool a oder b (oder c...) stammen, in einer oder in mehreren Fraktionen wandern und zur weiteren Untersuchung abgetrennt und gesammelt werden.

### 3. Quantitative Amplifizierung quantitative PCR

Die gesammelten Fraktionen werden einer quantitativen PCR unterworfen, wobei ein für alle Markierungssequenzen spezifisches Primer-Paar verwendet wird. Die quantitative PCR Reaktionslösung enthält zwei oder mehrere spezifische Fluoreszenz-Sonden alpha, beta (gamma...), die spezifisch mit den Markierungssequenzen A und B (C...) hybridisieren und unterschiedliche Emissionswellenlängen und daher Farben 1 und 2 (3...) aufweisen. In jeder Fraktion, in der sich mindestens ein Protein befindet, werden die Markierungssequenzen amplifiziert. Die Signalintensität des Amplifikates läßt auf die Menge der Markierungs-Sequenz in der untersuchten Fraktion schliessen, die wiederum eine Aussage über die Menge des darin befindlichen Proteins zuläßt. Anhand der Intensität der Farbe, die für die jeweilige Sonde spezifisch ist, wird darüber hinaus festgestellt, ob eine der Farben überwiegt.

### 4. Auswertung

Wenn die Farbe 1 überwiegt, heißt es, dass die Sonde alpha mit der Markierungssequenz A in höherem Masse hybridisiert als die Sonde beta mit der Markierungssequenz B. Daraus ergibt es sich, dass im Proteinpool a eins oder mehrere dieser Fraktion zugehörigen Proteine mehr vertreten waren als im Proteinpool b. Die Zustandsveränderung führte also zur Erniedrigung der Expression eines oder mehrerer Proteine in dieser Fraktion. Umgekehrt spricht das Überwiegen der Farbe 2 für eine Erhöhung der Expression von einem oder mehreren Proteinen aus dieser Fraktion nach der Zustandsveränderung.

### 5. Weiterverarbeitung

Da die Auftrennung/Fraktionierung ein definiertes und wiederholbares Vorgehen ist, läßt sich jede Fraktion, in der sich eine Expressionsveränderung abzeichnet, in ausreichender Menge gewinnen, und die darin befindlichen Proteine lassen sich isolieren und/oder mit präzisen Mitteln wie der Massenspektroskopie identifizieren.

### Ausführungsbeispiel:

Die zu untersuchenden Zellen werden in mindestens 2 Fraktionen (a, b, ...) aufgeteilt.

Es werden die Proteine von Zellen oder zellfreien Proteinsystemen mindestens einer Fraktion (a, ...) durch kovalente Bindung mindestens einer spezifischen Nukleinsäure-Sequenz (A) markiert.

Mindestens eine weitere Zellfraktion (b, ...) wird einer Zustandsveränderung ausgesetzt.

Die Proteine der Zellen oder zellfreien Proteinsysteme der Fraktion (b, ...) nach der Zustandsveränderung werden durch kovalente Bindung mindestens einer spezifischen Nukleinsäure-Sequenz (B) markiert, wobei die Sequenzen (A) und (B) identische 3'- und 5'-endständige Sequenzen, aber unterschiedliche Sequenzabschnitte im mittleren Bereich aufweisen.

Es erfolgt eine Vermischung der verschiedenen Proteinfraktionen (a, b, ...) und eine Auftrennung und Isolierung der Proteine des Fraktionsgemisches.

Die quantitative Bestimmung der gebundenen Nukleinsäure-Sequenzen erfolgt mittels quantitativer PCR wobei ein für alle Markierungssequenzen universales Primer-Paar zur Amplifikation verwendet wird und weiterhin spezifische Fluoreszenzsonden (alpha, beta, ...) in der quantitativen PCR-Reaktionslösung enthalten sind, die spezifisch mit den Markierungssequenzen (A, B, ...) hybridisieren und unterschiedliche Emissionswellenlängen aufweisen.

Die Fluoreszenzintensität der einzelnen Sonden wird bestimmt und verglichen, wobei eine größere Intensität der Sonde alpha, die spezifisch für die Markersequenz A ist, mit dem die Proteinfraktion a (Zellen vor der Zustandsänderung) markiert wurde, gegenüber einer geringeren Intensität des Sonde beta, die spezifisch für die Markersequenz B ist, mit dem die Proteinfraktion b (Zellen nach der Zustandsänderung) markiert wurde, eine Verringerung der Expression des untersuchten Poteins durch die Zustandsänderung der Zellen anzeigt bzw. eine größere Intensität der Sonde beta, die spezifisch für die Markersequenz B ist, mit dem die Proteinfraktion b (Zellen oder zellfreies System nach der Zustandsänderung) markiert wurde, gegenüber einer geringeren Intensität des Sonde alpha, die spezifisch für die Markersequenz A ist, mit dem die Proteinfraktion a (Zellen oder ein zellfreies System vor der Zustandsänderung) markiert wurde, eine Erhöhung der Expression des untersuchten Proteins durch die Zustandsänderung der Zellen anzeigt.

## Patentansprüche

1. Verfahren zur Erstellung von Profilen der differentialen Proteinexpression zum Vergleich von Proteomen, **dadurch gekennzeichnet, dass**
- die Proteome von Zellen vor und nach einer Zustandsänderung der Zellen jeweils mit Nukleinsäure-Sequenzen, welche identische 3'- und 5'-endständige Sequenzen, aber unterschiedliche Sequenzabschnitte im mittleren Bereich aufweisen, markiert werden;
- die mit den verschiedenen Nukleinsäure-Sequenzen markierten Proteome vermischt werden;
- eine Auftrennung und Isolierung der markierten Proteine erfolgt;
- und nachfolgend eine quantitative Bestimmung der Proteine durch quantitative Amplifikation der Markierungssequenzen erfolgt;
- eine Zuordnung der markierten Proteine zu den jeweiligen Proteomen unter Verwendung der Spezifischen Nukleinsäure - Markierungen der untersuchten Proteome erfolgt.

2. Verfahren nach Anspruch 1, **dadurch gekennzeichnet, dass** die Nukleinsäure-Sequenzen an mindestens einem 5'- oder 3'-Ende eine reaktive Gruppe aufweisen.

3. Verfahren nach Anspruch 2, **dadurch gekennzeichnet, dass** die reaktive Gruppe vorzugsweise eine NH₂- und/oder eine SH₂-Gruppe ist.

4. Verfahren nach Anspruch 1 - 3, **dadurch gekennzeichnet, dass** die Nukleinsäure-Sequenzen mit Hilfe sogenannter Crosslinker kovalent an ein Protein gebunden werde.

5. Verfahren nach Anspruch 1 - 4, **dadurch gekennzeichnet, dass** die Nukleinsäure-Sequenzen an eine NH₂-, COOH- und/oder SH-Gruppe eines Proteins kovalent gebunden werden.

6. Verfahren nach Anspruch 1 - 5, **dadurch gekennzeichnet, dass** als Crosslinker vorzugsweise N-Succinimidyl-(4-vinylsulfonyl)benzoate, Sulfosuccinimidyl-4-(P-Maleimidophenyl) Butyrate, Succinimidyl-4(N-maleimidomethyl) cyclohexane-1-carboxylate und/oder Sulfosuccinimidyl-4(N-maleimidomethyl)cyclohexane-1-carboxylate eingesetzt werden.

7. Verfahren nach Anspruch 1 - 6, **dadurch gekennzeichnet, dass** die Nukleinsäure-Sequenzen eine Länge von mindestens 45 Basenpaaren aufweisen.

8. Verfahren nach Anspruch 1 - 7, **dadurch gekennzeichnet, dass** die identischen 3'- und 5'endständigen Sequenzen eine Länge von 15 bis 25 Basenpaaren aufweisen

9. Verfahren nach Anspruch 1 - 8, **dadurch gekennzeichnet, dass** als identische 3'- und 5'endständige Sequenzen vorzugsweise folgende Sequenzen verwendet werden:

+Strang: 5´-GAGCATTCACGGTTCCTAAG.......................GAGTATCACGTTGCTAGTCG-3´
-Strang: 3´-CTCGTAAGTGCCAAGGATTC.......................CTCATAGTGCAACGATCAGC-5´

10. Verfahren nach Anspruch 1 - 9, **dadurch gekennzeichnet, dass** die unterschiedlichen Sequenzabschnitte im mittleren Bereich eine Länge von mindestens 15 Basenpaaren aufweisen.

11. Verfahren nach Anspruch 1 - 10, **dadurch gekennzeichnet, dass** die unterschiedlichen Sequenzabschnitte im mittleren Bereich eine Länge von 20 Basenpaaren aufweisen.

12. Verfahren nach Anspruch 1 - 11, **dadurch gekennzeichnet, dass** als unterschiedliche Sequenzabschnitte im mittleren Bereich folgende Sequenzen verwendet werden:

Sequenz A
+Strang: 5´-ACTTCACGTGCATTGGCACACTCGATGCAATTGATCAGGC. . .
-Strang: 3´-TGAAGTGCACGTAACCGTGTGAGCTACGTTAACTAGTCCG

Sequenz B
+Strang: 5´-CAATACAGAGACTACGACACATAGCAGACCTAGCTACGGA
-Strang: 5´-GTTATGTCTCTGATGCTGTGTATCGTCTGGATCGATGCCT

13. Verfahren nach Anspruch 1 - 12, **dadurch gekennzeichnet, dass** die Auftrennung der Proteine durch Chromatografie, ausgewählt aus lonenaustausch,- hydrophober Interaktions, "reverse phase", Permeationschromatografie, Immunaffinitäts-Chromatographie, Metall-Bindung-Chromatografie, Heparin-, und Gelatine-Chromatografie, oder durch chirale chromatografische Auftrennungsmethoden, Kapillar-Elektrophorese, Isotachophorese, isoelektrische Fokussierung oder klassische Akrylamid- oder Agarose-Elektrophorese erfolgt.

14. Verfahren nach Anspruch 1 - 13, **dadurch gekennzeichnet, dass** die quantitative Bestimmung der Nukleinsäure-Sequenzen vorzugsweise durch quantitative PCR erfolgt, wobei eine Unterscheidung zwischen den Sequenzen, mit denen das Proteom der Zellen vor der Zustandsänderung markiert wurde und den Sequenzen, mit denen das Proteom der Zellen nach der Zustandsänderung markiert wurde, durch die unterschiedlichen Sequenzabschnitte im mittleren Bereich ermöglicht wird.

15. Verfahren nach Anspruch 1 - 14, **dadurch gekennzeichnet, dass** die Amplifikation der Nukleinsäure-Sequenzen während der quantitative PCR Primern erfolgt, die komplementär zu den identischen 3'- und 5'-endständigen Sequenzen sind.

16. Verfahren nach Anspruch 1 - 15, **dadurch gekennzeichnet, dass** die Detektion der Nukleinsäure-Sequenzen während der quantitativen PCR mit Sonden erfolgt, die spezifisch an die unterschiedlichen Sequenzabschnitte im mittleren Bereich hybridisieren.

17. Verfahren nach Anspruch 1 - 16, **dadurch gekennzeichnet, dass** fluoreszenzmarkierte Sonden verwendet werden.

18. Verfahren nach Anspruch 1 - 17, **dadurch gekennzeichnet, dass** folgende Fluoreszenzmarkierungen verwendet werden: FAM, TAMRA, JOE, HEX, BIODIPY, Oregon Green, Rhodamine, Cy3, Cy5, CY5.5.

19. Verfahren nach Anspruch 1 - 18, **dadurch gekennzeichnet, dass** ein Vergleich der Mengen der proteingebundenen Nukleinsäure-Sequenzen der Zellen vor und nach der Zustandsänderung erfolgt, wobei eine erhöhte Konzentration der Sequenzen, mit denen das Proteom der Zellen oder ein zellfreies Proteinsystem vor der Zustandsänderung markiert wurde gegenüber einer geringeren Konzentration der Sequenzen, mit denen das Proteom der Zellen oder ein zellfreies Proteinsystem nach der Zustandsänderung markiert wurde, eine Verringerung der Expression des untersuchten Proteins durch die spezielle Zustandsänderung anzeigt bzw. eine erniedrigte Konzentration der Sequenzen, mit denen das Proteom der Zellen oder ein zellfreies Proteinsystem vor der Zustandsänderung markiert wurde gegenüber einer höheren Konzentration der Sequenzen, mit denen das Proteom der Zellen oder ein zellfreies Proteinsystem nach der Zustandsänderung markiert wurde, eine Erhöhung der Expression des untersuchten Proteins durch die spezielle Zustandsänderung anzeigt.

20. Verfahren nach Anspruch 1 -19, **dadurch gekennzeichnet, dass** eine Identifizierung der untersuchten Proteine durch Methoden, ausgewählt aus Massenspektroskopie, zweidimensionaler oder eindimensionaler Elektrophorese, und Aminosäuren-Sequenzierung erfolgt.

21. Verfahren nach Anspruch 1 - 20, **dadurch gekennzeichnet, dass**
- die zu untersuchenden Zellen in mindestens 2 Fraktionen (a, b, ...) aufgeteilt werden;
- eine Markierung der Proteine von Zellen oder zellfreien Proteinsystemen mindestens einer Fraktion (a, ...) durch kovalente Bindung mindestens einer spezifischen Nukleinsäure-Sequenz (A) an die Proteine erfolgt;
- mindestens eine weitere Zellfraktion (b, ...) einer Zustandsveränderung ausgesetzt wird;
- eine Markierung der Proteine der Zellen der Fraktion (b, ...) nach der Zustandsveränderung durch kovalente Bindung mindestens einer spezifischen Nukleinsäure-Sequenz (B) an die Proteine erfolgt, wobei die Sequenzen (A) und (B) identische 3'- und 5'-endständige Sequenzen, aber unterschiedliche Sequenzabschnitte im mittleren Bereich aufweisen;
- eine Vermischung der verschiedenen Proteinfraktionen (a, b, ...) erfolgt;
- eine Auftrennung und Isolierung der Proteine des Fraktionsgemisches erfolgt;
- die an den isolierten Proteinen gebundenen Nukleinsäure-Sequenzen einer quantitativen PCR unterworfen werden, wobei ein für alle Markierungssequenzen universales Primer-Paar zur Amplifikation verwendet wird und weiterhin spezifische Fluoreszenzsonden (alpha, beta, ...) in der quantitativen PCR-Reaktionslösung enthalten sind, die spezifisch mit den Markierungssequenzen (A, B, ...) hybridisieren und unterschiedliche Emissionswellenlängen aufweisen;
- die Fluoreszenzintensität der einzelnen Sonden bestimmt und verglichen wird, wobei eine größere Intensität der Sonde alpha, die spezifisch für die Markersequenz A ist, mit dem die Proteinfraktion a (Zellen vor der Zustandsänderung) markiert wurde, gegenüber einer geringeren Intensität der Sonde beta, die spezifisch für die Markersequenz B ist, mit dem die Proteinfraktion b (Zellen nach der Zustandsänderung) markiert wurde, eine Verringerung der Expression des untersuchten Poteins durch die Zustandsänderung der Zellen anzeigt bzw. eine größere Intensität der Sonde beta, die spezifisch für die Markersequenz B ist, mit dem die Proteinfraktion b (Zellen oder zellfreies System nach der Zustandsänderung) markiert wurde, gegenüber einer geringeren Intensität des Sonde alpha, die spezifisch für die Markersequenz A ist, mit dem die Proteinfraktion a (Zellen oder ein zellfreies System vor der Zustandsänderung) markiert wurde, eine Erhöhung der Expression des untersuchten Proteins durch die Zustandsänderung der Zellen anzeigt.

## Claims

1. Procedure for the production of profiles of the differential protein expression for the comparison of proteomes, wherein
- the proteomes of cells are marked with nucleic acid sequences manifesting identical 3' and 5' terminal sequences, but differing sequence sections in the middle area, before and after a change of state of the cells;
- the proteomes marked with the differing nucleic acid sequences are mixed;
- a separation and isolation of the marked proteins is done;
- and subsequently a quantitative determination of the proteins is done by quantitative amplification of the marking sequences;
- an assignment of the marked proteins to the proteomes in question is done making use of the specific nucleic acid markings of the proteomes examined.

2. Procedure according to Claim 1, wherein the nucleic acid sequences manifest a reactive group on at least one 5' or 3' end.

3. Procedure according to Claim 2, wherein the reactive group is preferably an NH₂ and/or an SH₂ group.

4. Procedure according to Claims 1 - 3, wherein the nucleic acid sequences are co-valently bound to a protein with the help of so-called cross-linkers.

5. Procedure according to Claims 1 - 4, wherein the nucleic acid sequences are co-valently bound to an NH₂, COOH and/or SH group of a protein.

6. Procedure according to Claims 1 - 5, wherein N-Succinimidyl-(4-vinylsulfonyl)benzoate, Sulfosuccinimidyl-4-(P-Maleimidophenyl) Butyrate, Succinimidyl-4(N-maleimidomethyl) cyclohexane-1-carboxylate and/or Sulfosuccinimidyl-4(N-maleimidomethyl)cyclohexane-1-carboxylate are preferably used as cross-linkers.

7. Procedure according to Claims 1 - 6, wherein the nucleic acid sequences manifest a length of at least 45 base pairs.

8. Procedure according to Claims 1 - 7, wherein the identical 3' and 5' terminal sequences manifest a length of 15 to 25 base pairs.

9. Procedure according to Claims 1 - 8, wherein the following sequences are preferably used as identical 3' and 5' terminal sequences:

+strain: 5´-GAGCATTCACGGTTCCTAAG......................GAGTATCACGTTGCTAGTCG-3´
-strain: 3´-CTCGTAAGTGCCAAGGATTC......................CTCATAGTGCAACGATCAGC-5´

10. Procedure according to Claims 1 - 9, wherein the various sequence sections in the middle area manifest a length of at least 15 base pairs.

11. Procedure according to Claims 1 - 10, wherein the various sequence sections in the middle area manifest a length of at least 20 base pairs.

12. Procedure according to Claims 1 - 11, wherein the following sequences are used as differing sequence sections in the middle area:

Sequence A
+strain: 5´-ACTTCACGTGCATTGGCACACTCGATGCAATTGATCAGGC
-strain: 3´-TGAAGTGCACGTAACCGTGTGAGCTACGTTAACTAGTCCG

Sequence B
+strain: 5´-CAATACAGAGACTACGACACATAGCAGACCTAGCTACGGA
-strain: 5´-GTTATGTCTCTGATGCTGTGTATCGTCTGGATCGATGCCT

13. Procedure according to Claims 1 - 12, wherein the fissure of the proteins is done by chromatography, selected from ion exchange, hydrophobic interaction, "reverse phase", permeation chromatography, immune affinity chromatography, metal binding chromatography, heparin and gelatine chromatography or by chiral chromatographic fissure methods, capillary electrophoresis, isotachophoresis, isoelectrical focusing or classical acrylic amide or agarosis electrophoresis.

14. Procedure according to Claims 1 - 13, wherein the quantitative determination of the nucleic acid sequences is preferably done by quantitative PCR, with a distinction between the sequences with which the proteome of the cells before the change of state was marked and the sequences with which the proteome of the cells after the change of state was marked being made possible by the differing sequence sections in the middle area.

15. Procedure according to Claims 1 - 14, wherein the amplification of the nucleic acid sequences during the quantitative PCR is done with primers complementary to the identical 3' and 5' terminal sequences.

16. Procedure according to Claims 1 - 15, wherein the detection of the nucleic acid sequences during the quantitative PCR is done with probes specifically hybridising to the differing sequence sections in the middle area.

17. Procedure according to Claims 1 - 16, wherein fluorescence-marked probes are used.

18. Procedure according to Claims 1 - 17, wherein the following fluorescence markings are used: FAM, TAMRA, JOE, HEX, BIODIPY, Oregon Green, Rhodamine, Cy3, Cy5, CY5.5.

19. Procedure according to Claims 1 - 18, wherein a comparison of the quantities of the protein-bound nucleic acid sequences of the cells is done before and after the change of state, with an increased concentration of the sequences with which the proteome of the cells or a cell-free protein system was marked before the change of state compared with a lower concentration of the sequences with which the proteome of the cells or a cell-free protein system was marked before the change of state displaying a reduction of the expression of the protein examined through the specific change of state or a reduced concentration of the sequences with which the proteome of the cells or a cell-free protein system was marked before the change of state compared with a higher concentration of the sequences with which the proteome of the cells or a cell-free protein system was marked after the change of state displaying an increase of the expression of the protein examined through the specific change of state, as the case may be.

20. Procedure according to Claims 1 - 19, wherein an identification of the examined proteins is done by methods selected from mass spectroscopy, two-dimensional or one-dimensional electrophoresis and amino acid sequencing.

21. Procedure according to Claims 1 - 20, wherein
- the cells to be examined are split into at least 2 fractions (a, b, ...);
- marking of the proteins of cells or cell-free protein systems of at least one fraction (a, ...) is done by co-valent binding of at least one specific nucleic acid sequence (A) to the proteins;
- at least one further cell fraction (b, ...) is subjected to a change of state;
- a marking of the proteins of the cells of fraction (b, ...) is done after the change of state by co-valent binding of at least one specific nucleic acid sequence (B) to the proteins, with sequences (A) and (B) manifesting identical 3' and 5' terminal sequences, but differing sequence sections in the middle area;
- there is a mixing of the various protein fractions (a, b, ...);
- there is a separation and isolation of the proteins of the fraction mixture;
- the nucleic acid sequences bound to the isolated proteins are subjected to a quantitative PCR, with a primer pair universal for all marking sequences being used for amplification and further specific fluorescence probes (alpha, beta, ...) being contained in the quantitative PCR reaction solution, which hybridise specifically with the marking sequences (A, B, ...) and manifesting differing emission wavelengths;
- the fluorescence intensity of the individual probes is determined and compared, with a larger intensity of the probe alpha, which is specific for the marker sequence A, with which the protein fraction a (cells before the change of state) was marked, compared with a lower intensity of the probe beta, which is specific for the marker sequence B, with which the protein fraction b (cells after the change of state) was marked, displaying a reduction of the expression of the examined protein by the change of state of the cells or a larger intensity of the probe beta, which is specific for the marker sequence B, with which the protein fraction b (cells or cell-free system after the change of state) was marked, compared with a lower intensity of the probe alpha, which is specific for the marker sequence A, with which the protein fraction a (cells or cell-free system before the change of state) was marked, displaying an increase of the expression of the examined protein by the change of state of the cells, as the case may be.

## Revendications

1. Procédé pour la fabrication de profils de l'expression de protéine différentielle pour la comparaison de protéomes, **se caractérisant par le fait que**
- les protéomes de cellules sont marqués avant et après une modification de l'état des cellules respectivement avec des séquences d'acide nucléique qui présentent des séquences de terminaisons 3' et 5' identiques mais de sections de séquences différentes dans la zone du milieu ;
- les protéomes marqués avec les différentes séquences d'acide nucléique sont mélangés ;
- une séparation et isolation des protéines marquées a lieu ;
- et par la suite une détermination quantitative des protéines a lieu par amplification des séquences de marquage ;
- une affectation des protéines marquées aux protéomes correspondants a lieu en employant les marquages d'acide nucléique spécifiques des protéomes analysés.

2. Procédé selon la revendication 1, **se caractérisant par le fait que** les séquences d'acide nucléique présentent un groupe réactif à l'une des terminaisons 5' ou 3' au moins.

3. Procédé selon la revendication 2, **se caractérisant par le fait que** le groupe réactif est de préférence un groupe NH₂ et/ou SH₂.

4. Procédé selon les revendications 1 - 3, **se caractérisant par le fait que** les séquences d'acide nucléique sont liées à une protéine en covalence à l'aide de dits crosslinker.

5. Procédé selon les revendications 1 - 4, **se caractérisant par le fait que** les séquences d'acide nucléique sont liées à un groupe NH₂, COOH et/ou SH d'une protéine.

6. Procédé selon les revendications 1 - 5, **se caractérisant par le fait que** l'on utilise en tant que crosslinker de préférence N-succinimidyl-(4-vinylsulfonyl)benzoate, sulfosuccinimidyl-4-(P-maleimidophenyl) butyrate, succinimidyl-4(N-maleimidomethyl) cyclohexane-1-carboxylate et/ou sutfosuccinimidyl-4(N-mateimidomethyl)cyclohexane-1-carboxylate.

7. Procédé selon les revendications 1 - 6, **se caractérisant par le fait que** les séquences d'acide nucléique présentent une longueur de 45 appariements de bases au moins.

8. Procédé selon les revendications 1 -7, **se caractérisant par le fait que** les séquences de terminaisons 3' et 5' identiques présentent une longueur de 15 à 25 appariements de bases au moins.

9. Procédé selon les revendications 1 - 8, **se caractérisant par le fait que** les séquences suivantes sont utilisées de préférence en tant que séquences de terminaisons 3' et 5' identiques :

Brin plus : 5´-GAGCATTCACGGTTCCTAAG.................GAGTATCACGTTGCTAGTCG-3´
Brin moins : 3´-CTCGTAAGTGCCAAGGATTC..............CTCATAGTGCAACGATCAGC-5´

10. Procédé selon les revendications 1 -9, **se caractérisant par le fait que** les différentes sections de séquences présentent dans la zone du milieu une longueur de 15 appariements de bases au moins.

11. Procédé selon les revendications 1 -10, **se caractérisant par le fait que** les différentes sections de séquences présentent dans la zone du milieu une longueur de 20 appariements de bases au moins.

12. Procédé selon les revendications 1 - 11, **se caractérisant par le fait que** les séquences suivantes sont employées en tant que différentes sections de séquences dans la zone du milieu :

Séquence A
Brin plus :
5´-ACTTCACGTGCATTGGCACACTCGATGCAATTGATCAGGC
Brin moins :
3´-TGAAGTGCACGTAACCGTGTGAGCTACGTTAACTAGTCCG

Séquence B
Brin plus :
5´-CAATACAGAGACTACGACACATAGCAGACCTAGCTACGGA
Brin moins :
5´-GTTATGTCTCTGATGCTGTGTATCGTCTGGATCGATGCCT

13. Procédé selon les revendications 1 - 12, **se caractérisant par le fait que** la séparation des protéines par chromatographie, que l'on pourra choisir entre la chromatographie par échange d'ions, par interaction hydrophobe, par « reverse phase », la chromatographie par perméation, la chromatographie par affinité immunitaire, la chromatographie par liaison du métal, à l'héparine et à la gélatine ou par des méthodes de séparation chromatographiques chirales, électrophorèse capillaire, isotachophorèse, focalisation isoélectrique ou l'électrophorèse classique à l'acrylamide ou agarose.

14. Procédé selon les revendications 1 - 13, **se caractérisant par le fait que** la détermination quantitative des séquences d'acide nucléique se fait de préférence par PCR quantitatif, au cours duquel il est possible de différencier entre les séquences avec lesquelles le protéome des cellules a été marqué avant la modification de l'état et les séquences avec lesquelles le protéome des cellules a été marqué après la modification de l'état, grâce aux différentes sections de séquence dans la zone du milieu.

15. Procédé selon les revendications 1 - 14, **se caractérisant par le fait que** l'amplification des séquences d'acide nucléique se fait pendant la PCR quantitative avec des amorces qui sont complémentaires aux séquences de terminaisons 3' et 5' identiques.

16. Procédé selon les revendications 1 - 15, **se caractérisant par le fait que** la détection des séquences d'acide nucléique se fait pendant la PCR quantitative avec des sondes qui hybrident de façon spécifique aux différentes sections de séquence dans la zone du milieu.

17. Procédé selon les revendications 1 - 16, **se caractérisant par le fait que** l'on utiliser des sondes marquées par fluorescence.

18. Procédé selon les revendications 1 - 17, **se caractérisant par le fait que** les marquages par fluorescence suivants sont utilisés : FAM, TAMRA, JOE, HEX, BIODIPY, Oregon Green, Rhodamine, Cy3, Cy5, CY5.5.

19. Procédé selon les revendications 1 - 18, **se caractérisant par le fait qu'**une comparaison des quantités de séquences d'acide nucléique liées à la protéine des cellules se fait avant et après la modification de l'état, en considération du fait qu'une concentration élevée de séquences avec lesquelles le protéome des cellules ou un système de protéines sans cellule a été marqué avant la modification de l'état, indique par rapport à une concentration peu importante des séquences avec lesquelles le protéome des cellules ou un système de protéine sans cellule a été marqué après la modification de l'état, une réduction de l'expression de la protéine analysée en raison de la modification de l'état spécifique voire qu'une réduction de la concentration des séquences avec lesquelles le protéome des cellules ou un système de protéine sans cellule a été marqué avant la modification de l'état indique par rapport à une concentration plus élevée des séquences avec lesquelles le protéome des cellules ou un système de protéine sans cellule a été marqué après la modification de l'état, une augmentation de l'expression de la protéine analysée en raison de la modification de l'état spécifique.

20. Procédé selon les revendications 1 - 19, **se caractérisant par le fait qu'**une identification des protéines analysées se fait au moyen de méthodes que l'on peut choisir entre la spectroscopie de masse, l'électrophorèse bidimensionnelle ou unidimensionnelle, et le séquençage des acides aminés.

21. Procédé selon les revendications 1 - 20, **se caractérisant par le fait que**
- les cellules à analyser sont divisées en 2 fractions au moins (a, b, ...);
- un marquage des protéines de cellules ou des systèmes de protéine sans cellule se fait au moins sur une fraction (a, ...) par covalence d'au moins une séquence d'acide nucléique spécifique (A) sur les protéines ;
- au moins une autre fraction de cellule (b, ...) a subi une modification d'état ;
- un marquage des protéines des cellules de la fraction (b, ...) est fait après la modification de l'état par covalence d'au moins une séquence d'acide nucléique spécifique (B) sur les protéines, les séquences (A) et (B) étant des séquences de terminaison 3' et 5' identiques mais présentant différentes sections de séquences dans la zone du milieu ;
- un mélange des différentes fractions de protéine (a, b ...) a lieu ;
- une séparation et isolation des protéines du mélange de fractions a lieu ;
- les séquences d'acide nucléique liées aux protéines isolées sont soumises à une PCR quantitative, en considération du fait qu'une paire d'amorces universelle pour toutes les séquences de marquage est utilisé pour l'amplification et par ailleurs des sondes à fluorescence spécifiques (alpha, bêta, ...) se trouvent dans la solution de réaction de la PCR quantitative, sondes qui hybrident de façon spécifique avec les séquences de marquage (A, B, ...) et présentent différentes longueurs d'ondes d'émission :
- l'intensité de fluorescence des différentes sondes est déterminée et comparée, en considération du fait qu'une plus grande intensité de la sonde alpha qui est spécifique pour la séquence du marqueur A avec lequel la fraction de protéine a (cellules avant la modification de l'état) a été marquée, indique par rapport à une intensité peu élevée de la sonde bêta qui est spécifique de la séquence du marquer B avec lequel la fraction de protéine b (cellules après la modification de l'état) a été marqué, une réduction de l'expression de la protéine analysée en raison de la modification de l'état des cellules voire qu'une plus grande intensité de la sonde bêta qui est spécifique pour la séquence du marqueur B avec lequel la fraction de protéine b (cellules ou système sans cellule après la modification de l'état) a été marquée, indique par rapport à une intensité peu élevée de la sonde alpha qui est spécifique de la séquence du marquer A avec lequel la fraction de protéine a (cellules ou système sans cellule avant la modification de l'état) a été marqué, une augmentation de l'expression de la protéine analysée en raison de la modification de l'état des cellules.
